# EUROPÄISCHE PATENTSCHRIFT

(11) **EP 2 099 382 B1**
(45) Veröffentlichungstag und Bekanntmachung des Hinweises auf die Patenterteilung: **27.03.2013**
(21) Anmeldenummer: 07856555.3
(22) Anmeldetag: 11.12.2007
(51) Int. Cl.: A61C 17/08, A61C 17/12, B04B 1/06, A61M 1/00, F04D 17/08, B08B 15/00

(54) **SAUGVORRICHTUNG FÜR DENTALE, MEDIZINISCHE UND INDUSTRIELLE ZWECKE**
SUCTION DEVICE FOR DENTAL, MEDICAL AND INDUSTRIAL PURPOSES
DISPOSITIF D'ASPIRATION POUR USAGES DENTAIRES, MÉDICAUX ET INDUSTRIELS

(30) Priorität: 12.12.2006 DE 102006058955
(43) Veröffentlichungstag der Anmeldung: 16.09.2009
(73) Patentinhaber: Dürr Dental AG, 74321 Bietigheim-Bissingen (DE)
(72) Erfinder: THOMS, Michael, 74321 Bietigheim-Bissingen (DE); SCHNEPF, Jürgen, 74080 Heilbronn (DE); HÄGELE, Andreas, 71384 Weinstadt (DE)
(74) Vertreter: Ostertag, Reinhard
(86) Internationale Anmeldenummer: PCT/EP2007/010806
(87) Internationale Veröffentlichungsnummer: WO 2008/071388

(56) Entgegenhaltungen:
- EP-A- 1 366 728
- WO-A-2007/112814
- DE-A1- 10 010 077
- DE-A1-102004 028 942
- US-A- 4 824 333

## Beschreibung

Die vorliegende Erfindung betrifft eine Saugvorrichtung für dentale, medizinische und industrielle Zwecke gemäß dem Oberbegriff des Anspruchs 1.

Derartige Saugvorrichtungen sind als kommerzielle Produkte bekannt. Sie dienen im dentalen Bereich der Unterdruckbeaufschlagung eines Speichelhebers und /oder einer Saugkanüle, über welche Wasser, Schleim, Blut und Bohrklein, darunter insbesondere Zahnhartgewebe und Amalgampartikel aus dem Mund eines Patienten abgesaugt werden.

Aus der DE 100 10 077 A1 ist eine derartige Saugvorrichtung bekannte bei der die rotierenden Teile des Radialgebläses und des Zentrifugalabscheiders auf einer gemeinsamen Wellenanordnung sitzen, welche durch einen Antriebsmotor angetrieben wird. Auf diese Weise werden die Gebläseräder des Radialgebläses und eine Leitwand einer Zentrifugaltrommel des Zentrifugalabscheiders immer mit der gleichen Drehzahl angetrieben.

In der EP 1 366 728 sind Saugmaschinen beschrieben, die mit Abscheidegeräten kombiniert werden. Unter den beschriebenen Einheiten befinden sich auch solche, bei denen das Sauggebläse und der Abscheider durch getrennte Motoren angetrieben werden.

Durch die vorliegende Erfindung soll eine Saugvorrichtung gemäß dem Oberbegriff des Anspruchs 1 so weitergebildet werden, dass die rotierenden Teile des Radialgebläses und die rotierenden Teile des Zentrifugalabscheiders mit unterschiedlichen Drehzahlen gedreht werden können, und die Saugvorrichtung trotzdem insgesamt kompakt ist und einen hohen Wirkungsgrad der Abscheidung und der Unterdruckerzeugung hat.

Diese Aufgabe ist erfindungsgemäß gelöst durch eine Saugvorrichtung mit den im Anspruch 1 angegebenen Merkmalen.

Die erfindungsgemäße Saugvorrichtung hat den Vorteil, dass das Radialgebläse und der Zentrifugalabscheider unabhängig voneinander angetrieben werden. Der Zentrifugalabscheider kann somit mit in der Regel deutlich geringeren Drehzahlen laufen als das Gebläse. Durch die Hintereinander-Anordnung der Antriebsmotoren ist die Saugvorrichtung insgesamt sehr kompakt aufgebaut. Die angegebene Ausbildung der Antriebseinheit kommt ohne Getriebe aus, wodurch die erfindungsgemäße Saugvorrichtung einfach und preisgünstig herzustellen ist und besonders kompakt baut.

Bei dem Radialgebläse der erfindungsgemäßen Saugvorrichtung ist gegenüber aus dem Stand der Technik bekannten Seitenkanalmaschinen die Druck/Volumen-Kennlinie deutlich flacher. Bei der erfindungsgemäßen Saugvorrichtung mit dem Radialgebläse ist der Leistungsbedarf proportional zur Luftmenge und nicht proportional zum Druck, wie dies bei den bekannten Seitenkanalmaschinen der Fall ist. Auf diese Weise ist die Abwärme bei allen Betriebspunkten geringer als bei herkömmlichen dentalen Saugmaschinen.

Sowohl das Radialgebläse als auch der Zentrifugalabscheider sind über die Steuereinheit in der Drehzahl steuer- und regelbar. Hierbei kann die jeweilige Drehzahl konstant vorgegeben werden. Dies ist dann bevorzugt, wenn beispielsweise bei einer Zahnbehandlung die Saugleistung der Saugvorrichtung variiert werden soll, um hierdurch Vorteile bei der Behandlung zu erzielen.

Darüber hinaus können auf diese Weise auch mehrere Arbeitsstationen parallel zueinander betrieben werden.

Alternativ kann die Drehzahl auch druckgeführt geregelt werden. Dies hat den Vorteil, dass ein konstanter Saugunterdruck erzielt werden kann, selbst wenn mehrere Arbeitsstationen parallel an der Saugvorrichtung angeschlossen sind, was zu stark wechselnden Saugluftanforderungen führen kann. Eine Konstanthaltung des Saugunterdrucks ist insbesondere bei der Zahnbehandlung oft erforderlich.

Die Verwendung des Radialgebläses hat darüber hinaus den Vorteil, dass dieses axial und radial größere Abstände zu stehenden Gehäuseteilen zulässt, als dies bei aus dem Stand der Technik bekannten herkömmlichen Saugmaschinen möglich ist. Dies erleichtert die Montage. Das Radialgebläse ist daher in Vergleich zu bekannten Seitenkanal-Vakuummaschinen auch deutlich weniger anfällig gegenüber Ablagerungen im Strömungsraum.

Dadurch, dass der Zentrifugalabscheider mit geringeren Drehzahlen betrieben werden kann als das Radialgebläse, kann der Antrieb in den wasserführenden Bereichen des Zentrifugalabscheiders mit deutlich geringeren Energieverlusten betrieben werden, als dies bei den höheren Drehzahlen des Radialgebläses der Fall ist.

Eine gehäusefeste Achse, auf der zwei Rotoren der beiden Antriebsmotoren gelagert sind 5Anspruch 1°, kann dabei als tragendes Teil dienen, so dass diese umgebende Gehäuseteile aus leichtem Material, beispielsweise Kunststoff, gefertigt werden können. Eine gemeinsame Lagerachse hat auch den Vorteil eines besonders kompakten und robusten Gesamtaufbaus, der sich mit geringem Aufwand montieren läßt.

Die Anordnung von Rotorglocken jeweils an den Enden der gehäusefesten Achse gemäß Anspruch 3 ist besonders platzsparend.

Elektronisch kommutierte Antriebsmotoren gemäß Anspruch 4 können mit hohen Drehzahlen betrieben werden,und haben einen guten Wirkungsgrad bei geringer Stromaufnahme. Elektronisch kommutierte Antriebsmotoren können ferner mit innerhalb von gewissen Grenzen variablen Eingangsnetzspannungen betrieben werden, was bei aus dem Stand der Technik bekannten Asynchronmotoren nicht möglich ist. Sie können bei unterschiedlichen Netzfrequenzen und Netzspannungen eingesetzt werden, ohne dass unterschiedliche Rotorausführungen erforderlich sind. Auf diese Weise wird der Fertigungsaufwand und der Lieferaufwand deutlich reduziert.

Die Verwendung von Magnetfeldgebern auf Kommutatorplatinen gemäß Anspruch 5 ermöglicht die Realisierung von verschleißfreien Kommutatoren.

Als besonders günstig im Hinblick auf den mit der Saugvorrichtung erzeugbaren Druck hat sich eine Saugvorrichtung gemäß Anspruch 6 herausgestellt, bei der das Radialgebläse zweistufig ist. Mit einem zweistufigen Radialgebläse können größere Drucke erzeugt werden als mit einstufigen Gebläsen. Hingegen hat ein zweistufiges Radialgebläse gegenüber drei- oder mehrstufigen Radialgebläsen den Vorteil, dass die Druckverluste auf Grund von Luftumlenkungen innerhalb des Gehäuses des Radialgebläses geringer sind.

Mit einem Lüfterrad gemäß Anspruch 7 können die wärmebelasteten Bauteile im Inneren der Saugvorrichtung wirksam gekühlt werden. Auf diese Weise ist ein Dauereinsatz der Saugvorrichtung realisierbar, was besonders im zahnmedizinischen Bereich, insbesondere in einer Zahnarztpraxis oder einer Zahnklinik, erforderlich ist. Die Lebensdauer der wärmebelasteten Bauteile wird dadurch erhöht und die Saugvorrichtung kann auch mit hohen Gebläsedrehzahlen dauerhaft in den oben genannten Einsatzbereichen verwendet werden. Die Anordnung des Lüfterrades zwischen den beiden Antriebsmotoren koaxial ist platzsparend, außerdem hat der Antrieb durch einen der beiden Antriebsmotoren den Vorteil, dass kein separater Lüfterradmotor erforderlich ist.

Ein Hohlraum gemäß Anspruch 8 in der gehäusefesten Achse im Bereich von Lagern ermöglicht die Durchleitung von Kühlluft und somit eine Innenkühlung der Achse und der damit verbundenen Lager. Auf diese Weise wird eine Überhitzung der Lager bei hohen Drehzahlen verhindert und so deren Lebensdauer vergrößert.

Eine Steuereinheit kann gemäß Anspruch 9 besonders platzsparend in der Saugvorrichtung integriert werden. Dies hat gegenüber marktbekannten Saugvorrichtungen den Vorteil, dass keine externen Steuerkästen mit Schutzeinrichtungen und Überstromauslösern erforderlich sind, so dass der Montageaufwand und die Kosten deutlich geringer sind. Mit einer Steuereinheit für elektronisch kommutierte Antriebsmotoren kann darüber hinaus eine Strombegrenzung einfacher realisiert werden als mit einem Überstromschutz, der bei aus dem Stand der Technik bekannten Drehstrommotoren eingesetzt wird, zumal bei Drehstrommotoren für jede der drei Phasen ein Überstromschutz vorgesehen sein muß.

Ein Ausführungsbeispiel der Erfindung wird nachfolgend anhand der Zeichnung näher erläutert; es zeigen
- Figur 1: schematisch einen axialen Schnitt eines dentalen Sauggebläses mit einem integrierten Radialgebläse und einem Zentrifugalabscheider für angesaugte flüssige und feste Bestandteile, wobei der Übersichtlichkeit nur die Bezugszeichen für die wichtigsten Kompo nenten eingetragen sind;
- Figur 2: schematisch eine Seitenansicht des dentalen Sauggebläses aus Figur 1;
- Figur 3: schematisch eine isometrische Darstellung des dentalen Sauggebläses aus den Figuren 1 2;
- Figur 4: ein schematisches Blockschaltbild des dentalen Sauggebläses aus den Figuren 1 bis 3;.
- Figur 5: einen axialen Schnitt durch ein Radialgebläse des Sauggebläses von Figur 1 und seinen Antriebsmotor;
- Figur 6: einen axialen Schnitt durch eine mittlere Elektronik- und Lüftereinheit des Sauggeblä ses nach Figur 1 und den Antriebsmotor für Zentrifugalabscheider des Sauggebläses nach Figur 1;
- Figur 7: einen axialen Schnitt des Zentrifugalabscheiders des Sauggebläses nach Figur 1; und
- Figur 8: eine einstellbare Drossel für einen durch das

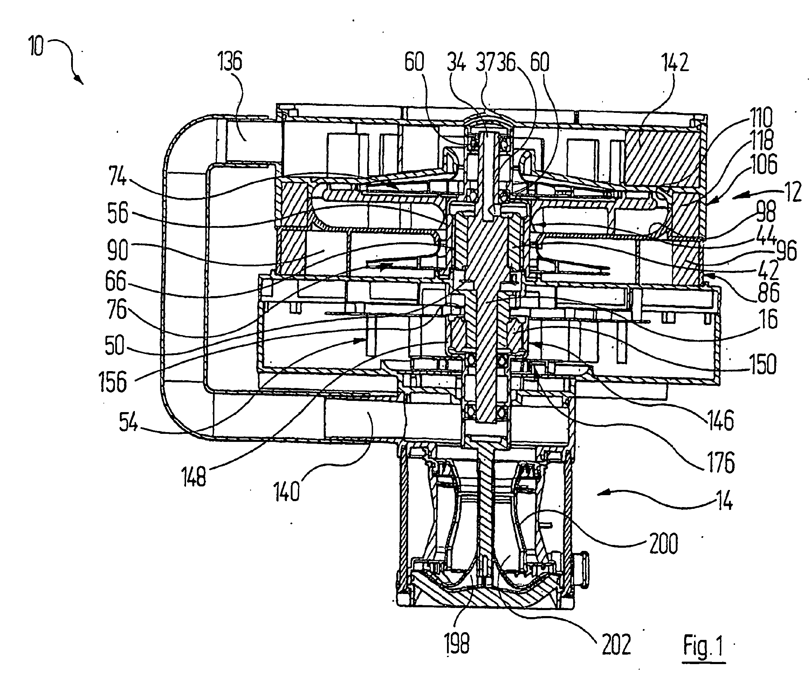

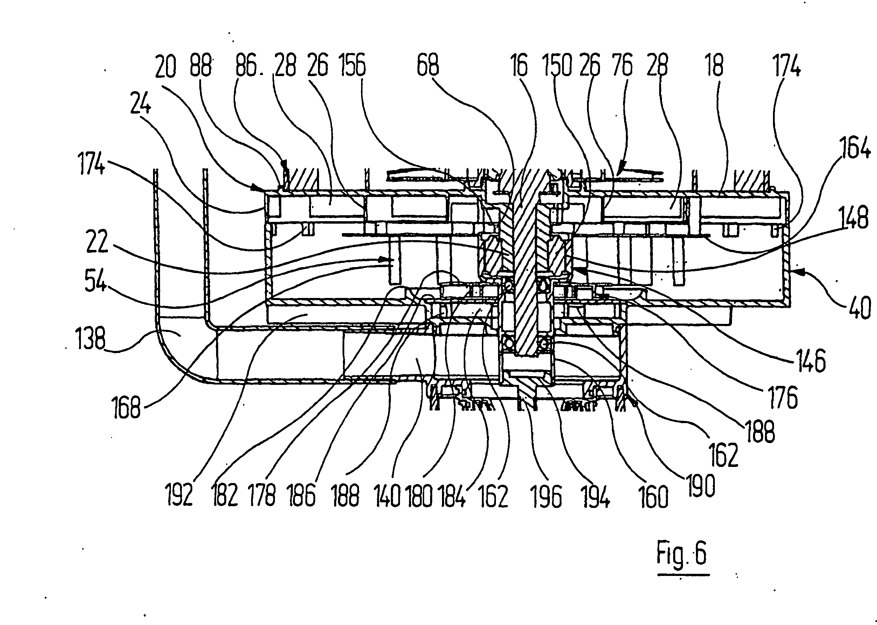

In den Figuren 1 bis 7 ist ein insgesamt mit dem Bezugszeichen 10 versehenes dentales Sauggebläse gezeigt. Das Sauggebläse 10 umfasst ein Radialgebläse 12, in Figur 1 bis 3 oben, mit dem ein Zentrifugalabscheider 14, in Figuren 1 bis 3 unten, fest verbunden ist.

Das Radialgebläse 12 und der Zentrifugalabscheider 14 sind bezüglich einer gehäusefesten Achse 16, welche sich in Figur 1 vertikal erstreckt, im Wesentlichen rotationssymmetrisch und koaxial zur gehäusefesten Achse 16 hintereinander angeordnet.

Die gehäusefeste Achse 16 führt mittig durch eine kreisrunde ebene Trägerplatte 18 eines insgesamt mit dem Bezugszeichen 20 versehenen Trägergehäuses. Die gehäusefeste Achse 16 ist in eine rotationssymmetrische, einstückig an die Trägerplatte 18 angeformte Tragbuchse 22 eingeschrumpft, welche sich auf der dem Zentrifugalabscheider 14 zugewandten Seite der Trägerplatte 18 befindet.

An den Rand der Trägerplatte 18 ist umlaufend eine zylindrische Umfangswand 24 angeformt, die zum Zentrifugalabscheider 14, also nach unten hin offen ist.

Auf der dem Zentrifugalabscheider 14 zugewandten Seite sind darüber hinaus zwei kreiszylindrische, zur gehäusefesten Achse 16 koaxiale Verstärkungsrippen 26 an die Trägerplatte 18 angeformt. Die axiale Ausdehnung der Verstärkungsrippen 26 entspricht der axialen Ausdehnung der Umfangswand 24. Zwischen den kreisförmigen Verstärkungsrippen 26 beziehungsweise zwischen der äußeren Verstärkungsrippe 26 und der Umfangswand 24 erstreckt sich eine Vielzahl von radial verlaufenden Querrippen 28.

Das Trägergehäuse 20 ist nach einem Druckgussverfahren aus Aluminium gefertigt. Es kann aber auch in anderer Weise hergestellt sein.

Insgesamt bilden das Trägergehäuse 20 und die gehäusefeste Achse 16 ein stabiles Skelett für das dentale Sauggebläse 10.

Auf der dem Zentrifugalabscheider 14 abgewandten Seite der Trägerplatte 18 befindet sich das Radialgebläse 12. Dort weist die gehäusefeste Achse 16 einen Stator-Tragbereich 30 mit einem erweiterten Querschnitt auf. Der Stator-Tragbereich 30 befindet sich in axialer Richtung in einem Abstand zu der oberen Stirnfläche der Tragbuchse 22. Der Außendurchmesser des Stator-Tragbereich 30 entspricht dem Außendurchmesser der Tragbuchse 22.

Die oberen Stirnfläche der Tragbuchse 22 bildet den Boden einer kreiszylindrischen, zur gehäusefesten Achse 16 koaxialen Motorkammer 32, die sich im Zentrum der Trägerplatte 18 befindet. Die Umfangsseite der Motorkammer 32 geht etwa in ihrer axialen Mitte einstückig in die Trägerplatte 18 über. Der Stator-Tragbereich 30 der gehäusefesten Achse 16 erstreckt sich in axialer Richtung etwa über ein Viertel der Gesamtlänge der gehäusefesten Achse 16, also etwa über die Hälfte der axialen Höhe des Radialgebläses 12.

Die gehäusefeste Achse 16 verfügt im Anschluss an den Stator-Tragbereich 30 über eine zylindrische axiale Bohrung 34, die zu der vom Zentrifugalabscheider 14 abgewandten Stirnseite der gehäusefesten Achse 16 hin offen ist. Etwas hinter dem Stator-Tragbereich 30 weist die gehäusefeste Achse 16 eine Querbohrung 36 auf, die von der Achsen-Mantelfläche zu der Bohrung 34 führt.

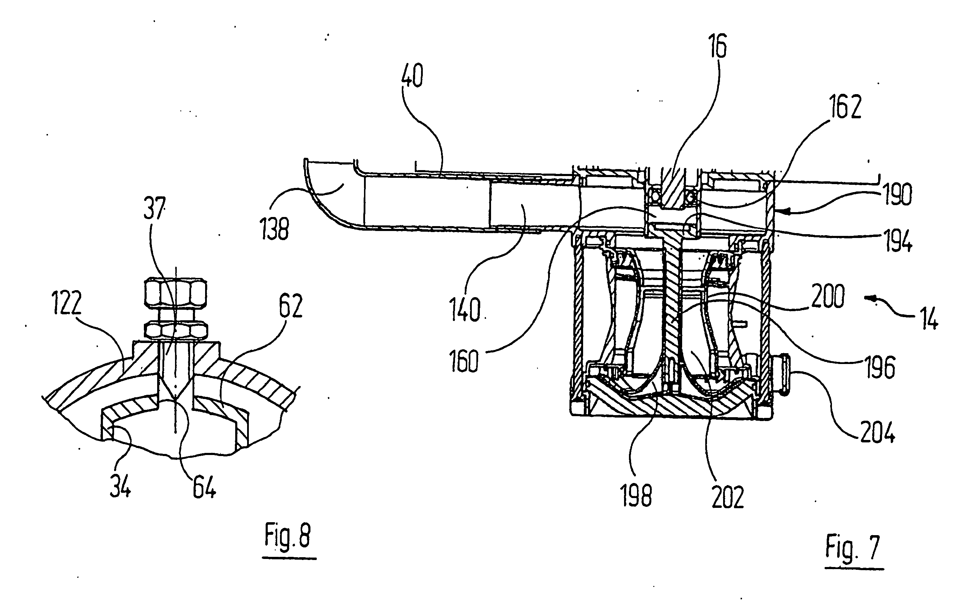

Durch eine Drosselschraube 37, die mit einer über dem Ende der Bohrung 34 liegenden Öffnung 64 zusammenarbeitet (Fig. 8), kann ein Kühlluftstrom durch das Innere des oberen Endabschnittes der Welle 16 eingestellt werden, dessen Erzeugung weiter unten beschrieben wird.

Der Boden der kreiszylindrischen Motorkammer 32 weist im Übrigen eine Mehrzahl durchgängiger Luftschlitze 38 auf. Durch die Luftschlitze 38 kann Luft aus einem Elektronikgehäuse 40, welches sich auf der dem Zentrifugalabscheider 14 zugewandten Seite der Trägerplatte. 18 befindet, in die Motorkammer 32 strömen.

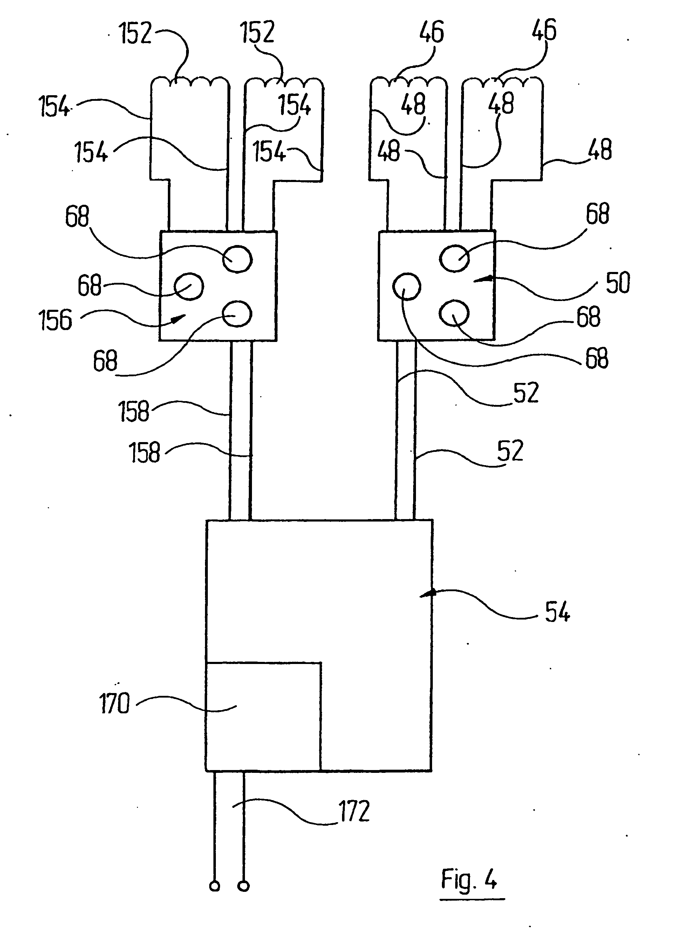

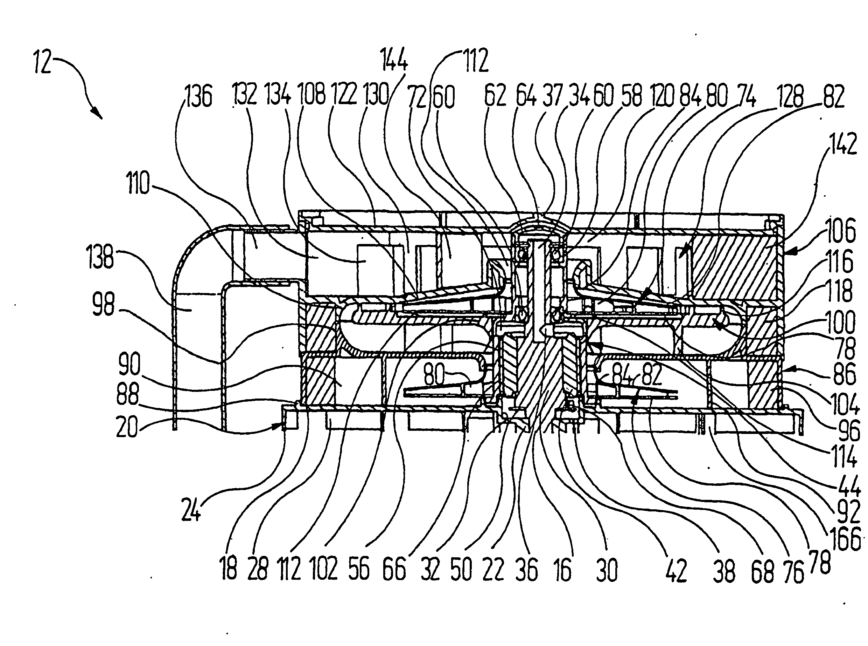

Auf der Umfangsfläche des Stator-Tragbereichs 30 der gehäusefesten Achse 16 sitzen einige Statorpakete 42, welche Teil eines insgesamt mit 44 bezeichneten Gebläse-Antriebsmotors ist. Die Statorpakete 42 umfassen eine Mehrzahl von Blechen. Die Bleche tragen in Umfangsrichtung verteilte, in Figur 4 abgebildete Wicklungen 46. Beispielhaft sind in der Figur 4 lediglich zwei der Wicklungen 46 gezeigt, die über jeweilige Verbindungsleitungen 48 mit einer Kommutatorplatine 50 verbunden sind. Die Kommutatorplatine 50 ist über zwei Versorgungsleitungen 52 jeweils mit einem Pol eines Spannungsausgangs einer Steuereinheit 54 verbunden. Bei entsprechender Strombeaufschlagung der Wicklungen 46 durch die Steuereinheit 54 erzeugen die Statorpakete 42 ein Drehfeld.

Auf der Gebläseseite ist auf der gehäusefesten Achse 16 eine Rotorglocke 56 aus Stahl für den Gebläse-Antriebsmotor 44 drehbar angeordnet. Die Rotorglocke 56 dreht sich in axialer Richtung zum Zentrifugalabscheider 14 hin betrachtet im Uhrzeigersinn.

An die Rotorglocke 56 ist auf der vom Zentrifugalabscheider 14 abgewandten Seite koaxial zur Rotorglocke 56 ein kreiszylindrisches Lagergehäuse 58 mit einem geringeren Durchmesser als dem der Rotorglocke 56 angeformt. In dem Lagergehäuse 58 sind zwei axial beabstandete Lager 60 angeordnet, über die die Rotorglocke 56 im Bereich des oberen Endes der gehäusefesten Achse 16 gelagert ist.

Die obere Stirnseite des Lagergehäuses 58 des Gebläse-Antriebsmotors 44 ist mit einer von außen betrachtet konvexen Kappe 62 verschlossen. Die Kappe 62 weist mittig eine Öffnung 64 für Kühlluft auf, welche stirnseitig aus der Bohrung 34 der gehäusefesten Achse 16 austritt. Durch die Kappe 62 sind die Lager 60 des Gebläse-Antriebsmotors 44 weitgehend gekapselt. Die Öffnung 64 ermöglicht ein dosiertes Abströmen der Kühlluft und stellt eine gute Kühlung der Lager 60 sicher.

Die Rotorglocke 56 ist zum Zentrifugalabscheider 14 hin offen. Sie trägt auf ihrer innenliegenden Umfangsfläche eine Mehrzahl in Umfangsrichtung verteilter Hochleistungsmagnetsegmente 66 oder auch einen Magnetring, der mit einer Vielzahl in Umfangsrichtung alternierender Magnetisierungen versehen ist.

Auf der dem Zentrifugalabscheider 14 zugewandten radialen Kreisringfläche des Stator-Tragbereichs 30 sitzt ferner die Kommutatorplatine 50 mit drei Hallgebern 68, mit der in bekannter Weise der Gebläse-Antriebsmotor 44 elektrisch kommutiert betrieben wird.

Die Kommutatorplatine 50 des Gebläse-Antriebsmotors 44 ist seitlich ausgespart, so dass Kühlluft, welche durch die Luftschlitze 38 im Bereich der Tragbuchse 22 aus dem Elektronikgehäuse 40 einströmt, an der Kommutatorplatine 50 vorbei zu den Statorpaketen 42 strömen kann. Darüber hinaus werden dort auch die Versorgungsleitungen 52 von der Steuereinheit 54 durchgeführt.

Die Rotorglocke 56 für den Gebläse-Antriebsmotor 44 ist so auf der gehäusefesten Achse 16 angeordnet, dass zwischen den Statorpaketen 42 und der dem Lagergehäuse 58 der Rotorglocke 56 zugewandten radialen Kreisringfläche ein Ringspalt 72 verbleibt. Mit dem Ringspalt 72 kommuniziert die Querbohrung 36, die von der Bohrung 34 der gehäusefesten Achse 16 kommt.

Zwischen den Statorpaketen 42 und den Hochleistungsmagnetsegmenten 66 befindet sich, wie gesagt ein schmaler Ringspalt, durch den Kühlluft in axialer Richtung strömen kann.

Auf dem Lagergehäuse 58 der Rotorglocke 56 sitzt ein erstes Laufrad 74 des Radialgebläses 12, auf dem unteren Ende der Rotorglocke 56 ein zweites Laufrad 76. Die Laufräder 74 und 76 sind jeweils über drei in den Figuren 1 bis 3 nicht gezeigte Metallschrauben mit der Umfangswand der Rotorglocke 56 beziehungsweise dem Lagergehäuse 58 verschraubt. Die Metallschrauben sind mit Kleber gesichert.

Die Laufräder 74 und 76 umfassen jeweils eine ebene, hochfeste Radscheibe 78, in Figur 1 unten, mit angeformten, entgegen der Drehrichtung der Laufräder 74 und 76 konkav gekrümmten Radschaufeln 80. Die Radscheiben 78 sind aus Kunststoff spritzgegossen und glasfaserverstärkt. Wegen ihrer Krümmung sind die Radschaufeln 80 strömungsgünstig und leise.

Die von der Radscheibe 78 abgelegenen Ränder der Radschaufeln 80 sind jeweils durch eine Stirnscheibe 82 abgedeckt, welche sich in radialer Richtung bis zu den äußeren Rändern der Radschaufeln 80 und den mit diesen radial fluchtenden äußeren Rändern der Radscheiben 78 erstrecken. In radialer Einwärtsrichtung überragen die Stirnscheiben 82 die radial inneren Enden der Radschaufeln 80.

Die Stirnscheiben 82 sind aus einer harten Aluminiumlegierung mit geringer Dichte. Alternativ können die Stirnscheiben 82 aus Kunststoff sein, welcher den gleichen Ausdehnungskoeffizient wie der Kunststoff hat, aus dem die Radscheiben 78 gefertigt sind.

Die Stirnscheiben 82 sind mittels Warmnieten oder Ultraschallnieten mit den Radschaufeln 80 verbunden.

Am radial innen liegenden Rand haben die Stirnscheiben 82 einen axial kurzen Einlassstutzen 84, der mit einer großzügig bemessenen Außenbiegung in die axial äußere Fläche übergeht, die gegenüber der Radscheibe 78 geneigt, also kegelstumpfförmig ist. In radial nach außen läuft die Stirnscheibe 82 auf die Radscheibe 78 zu.

Das zweite Laufrad 76 befindet sich in einer Laufradkammer im Zentrum eines becherförmigen Gehäusesegmentes 86, welches zur Trägerplatte 18 hin offen ist und in seinem Deckelteil eine Öffnung für die Rotorglocke 56 hat. Der Außendurchmesser des Gehäusesegmentes 86 ist etwas kleiner als der Außendurchmesser der Trägerplatte 18. Die Umfangswand des Gehäusesegmentes 86 liegt dicht an ihrem freien Rand mit ihrer äußeren Umfangsfläche an der Innenseite eines kreisförmigen Positioniersteges 88 an. Der Positioniersteg 88 ist einstückig an die dem Radialgebläse 12 zugewandte Oberfläche der Trägerplatte 18 angeformt.

In dem Gehäusesegment 86 fördert das zweite Laufrad 76 Luft in einen spiralförmigen, Auslasskanal 90, der durch einen spiralförmigen sich in Auslassrichtung erweiternden Wandabschnitt am.Rand des Gehäusesegmentes 86 und durch eine spiralförmige Randwand 92 begrenzt ist. Die Spiralform des Auslasskanals 90 ist logarithmisch. Der Auslasskanal 90 kann aber auch in anderer Weise so gestaltet sein, dass die ihn durchströmende Luft einen konstanten Drall hat.

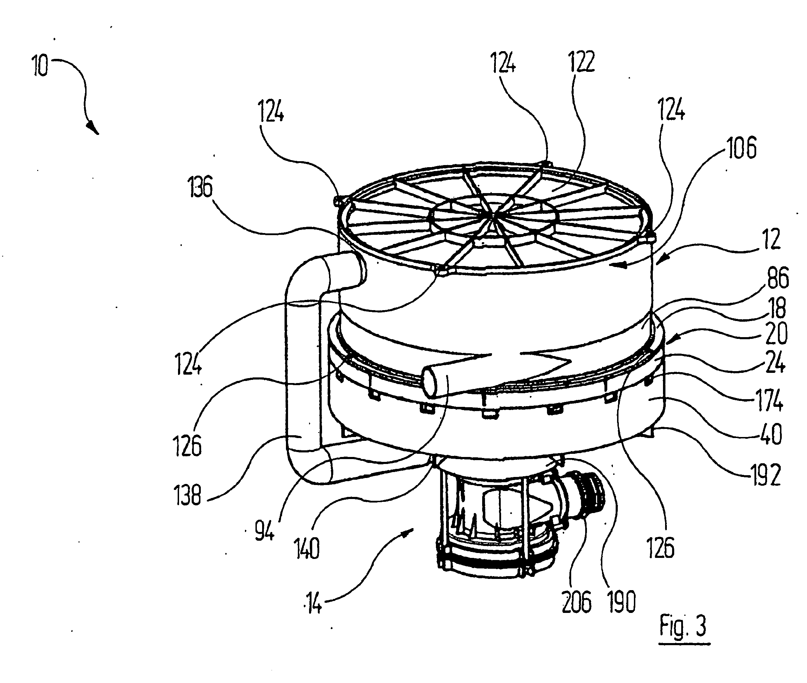

Der Wandabschnitt steht mit einem zum Auslasskanal 90 tangentialen Auslassstutzen 94 (vgl. Figur 3) in Verbindung. Die Randwand 92 hängt von dem oberen Deckelteil des Gehäusesegmentes 86 herab.

Ein Zwischenraum zwischen dem spiralförmigen Auslasskanal 90 und der radial äußeren Seitenwand des Gehäusesegmentes 86 ist mit einem Dämmschaum 96 zur Schalldämmung gefüllt.

Der radial innere Rand des oberen Deckelteils des Gehäusesegmentes 86 ist großzügig um 90° axial nach innen zur Trägerplatte 18 hin gebogen. Er überlappt in axialer Richtung radial von außen den Einlassstutzen 84 des zweiten Laufrads 76 unter Beibehaltung eines Laufspiels.

An die von der Trägerplatte 18 abgewandte Oberseite des oberen Deckelteils des Gehäusesegmentes 86 ist eine zur gehäusefesten Achse 16 rotationssymmetrische untere Umlenkstufenwand 98 angeformt.

Die radial von innen betrachtet konkav gewölbte Innenseite der unteren Umlenkstufenwand 98 hat im axialen Schnitt die Form eines Viertelkreises. Die Oberseite des oberen Deckelteils des Gehäusesegmentes 86 geht tangential in die Innenseite der unteren Umlenkstufenwand 98 über.

Der vom Gehäusesegment 86 abgewandte obere Rand der unteren Umlenkstufenwand 98 liegt in axialer Richtung etwas unterhalb der Kreisringfläche zwischen dem Lagergehäuse 58 und der Rotorglocke 56 des Gebläse-Antriebsmotors 44. Die radial äußere Wand der unteren Umlenkstufenwand 98 verläuft kreiszylindrisch koaxial zur gehäusefesten Achse 16. Sie fluchtet in axialer Richtung etwa mit der radial äußeren Begrenzung des spiralförmigen Auslasskanals 90 im Gehäusesegment 86.

In radialer Richtung fluchtet mit dem oberen Rand der unteren Umlenkstufenwand 98 eine der Trägerplatte 18 zugewandte Unterseite eines zur gehäusefesten Achse 16 rotationssymmetrischen, ebenen Leitelements 100. Der Rand des Leitelements 100 ist im axialen Schnitt strömungsgünstig halbreisbogenförmig abgetragen. Der Durchmesser des Leitelements 100 entspricht etwa dem Innendurchmesser der unteren Umlenkstufenwand98 im Bereich ihres tangentialen Übergangs zur Oberseite des oberen Deckelteils des Gehäusesegmentes 86.

In seinem Zentrum verfügt das Leitelement 100 über eine durchgängige, zur gehäusefesten Achse 16 rotationssymmetrische Öffnung für die Rotorglocke 56. An die Öffnung schließt sich auf der der Trägerplatte 18 zugewandten Unterseite des Leitelements 100 ein Kragen 102 an, dessen kreiszylindrische, zur gehäusefesten Achse 16 koaxiale Innenfläche die Rotorglocke 56 unter Beibehaltung eines Laufspiels umgibt. Die in radialer Richtung äußere Fläche des Kragens 102 geht strömungsgünstig gebogen, im Axialschnitt in Form eines Viertelkreisbogens, in die horizontale, dem Gehäusesegment 86 zugewandte Unterseite des Leitelements 100 über.

Von der Unterseite des Leitelements 100 hängen an diese angeformte Drallschaufeln 104 herab, die entgegen der Krümmung der Radschaufeln 80 der Laufräder 74 und 76 gekrümmt sind.

Die von dem Leitelement 100 abgelegenen Ränder der Drallschaufeln 104 liegen spaltfrei am ebenen Bereich der Oberseite des Deckelteils des Gehäusesegmentes 86 an. Die radial inneren Ränder der Drallschaufeln 104 reichen bis zum Übergang des ebenen Bereichs der Unterseite des Leitelements 100 in die Biegung der äußeren Fläche des Kragens 102.

Auf dem Gehäusesegment 86 sitzt ein zur gehäusefesten Achse 16 rotationssymmetrisches Ansaug-Gehäusesegment 106. Der Außendurchmesser des Ansaug-Gehäusesegments 106 entspricht in etwa dem Außendurchmesser des Gehäusesegmentes 86. Der dem Gehäusesegment 86 zugewandte Rand der Außenwand des Ansaug-Gehäusesegments 106 weist eine radial innenliegende Stufe auf, welche den oberen Rand des Gehäusesegmentes 86 übergreift und so das Ansaug-Gehäusesegment 106 gegenüber dem Gehäusesegment 86 in radialer Richtung verrutschsicher gehalten ist.

Ein Zwischenboden 108 des Ansaug-Gehäusesegments 106 befindet sich auf der dem Gehäusesegment 86 gegenüberliegenden Seite des Leitelements 100 und begrenzt mit diesem eine Laufradkammer, in der sich das erste Laufrad 74 dreht.

An die dem Gehäusesegment 86 zugewandte Unterseite des Zwischenbodens 108 ist eine obere Umlenkstufenwand 110 einstückig angeformt, deren freier Rand an dem freien Rand der unteren Umlenkstufenwand 98 dicht anliegt. Die Kontur der radial inneren Fläche der unteren Umlenkstufenwand 98 geht stufenlos in die entsprechende Kontur der oberen Umlenkstufenwand 110 über. Die radial innere Fläche der oberen Umlenkstufenwand 110 ist analog zur unteren Umlenkstufenwand 98 im Axialschnitt entsprechend eines Viertelkreisbogens gekrümmt, dessen Radius allerdings deutlich kleiner ist als der Radius des entsprechenden Viertelkreisbogens der unteren Umlenkstufenwand 98. Die Umlenkstufenwände 98 und 110 bilden zusammen eine Umlenkstufe, welche Luft vom radial außenliegenden Abgabebereich der ersten Gebläsestufe zum radial innenliegenden Ansaugbereich der zweiten Gebläsestufe führt.

Die radial inneren Fläche der oberen Umlenkstufenwand 110 geht tangential in einen kreisringförmigen horizontalen Bereich des Zwischenbodens 108 des Ansaug-Gehäusesegments 106 über.

In radialer Richtung etwa fluchtend mit den äußeren Rändern des ersten Laufrads 74 schließt sich an den kreisringförmigen horizontalen äußeren Bereich des Zwischenbodens 108 in radialer Einwärtsrichtung ein etwa kegelförmiger Bereich an, der in radialer Richtung zur gehäusefesten Achse 16 hin ansteigt. Der kegelförmige Bereich des Zwischenbodens 108 ist etwas steiler angestellt als die Stirnscheibe 82 des ersten Laufrads 74.

In seinem Zentrum weist der kegelförmige Bereich des Zwischenbodens 108 eine Durchlassöffnung für den Einlassstutzen 84 des ersten Laufrads 74 auf.

Der radial innere Rand des Zwischenbodens 108 ist zu einem Einlasstrichter 112 geformt. Der Zwischenboden 108 ist hierzu zunächst kreiszylinderförmig von der Trägerplatte 18 weg gebogen und oberhalb des freien Randes des Einlassstutzens 84 mit einer großzügigen Biegung trichterförmig nach unten zurück in Richtung auf die Trägerplatte 18 zu geführt. Der kreiszylinderförmige innere Randbereich des Zwischenbodens 108 umschließt den Einlassstutzen 84 des ersten Laufrads 74 unter Beibehaltung eines Laufspiels.

Das Leitelement 100 weist in seiner dem ersten Laufrad 74 zugewandten Oberfläche ferner eine flache, in Aufsicht kreisförmige Vertiefung 112 für die Radscheibe 78 des ersten Laufrads 74 auf, die über dessen äußeren Rand in radialer Richtung hinausgeführt ist. In radialer Richtung etwa mit der Mitte der gebogenen Fläche des Kragens 102 auf der Unterseite des Leitelements 100 fluchtend ist in der Vertiefung 112 ein axial nach oben vorstehender umlaufender Steg 114 angeformt. Der Steg 114 reicht unter Beibehaltung eines Laufspiels bis zur Stirnscheibe 82 des ersten Laufrads 74.

Auf der Oberseite des Leitelements 100 im Bereich außerhalb der Vertiefung 112 ist ferner ein Leitkranz angeordnet, welcher das erste Laufrad 74 umgibt. Der Leitkranz besteht aus einer Vielzahl von gehäusefesten Leitschaufeln 116, die die gehäusefeste Achse 16 nicht schneidende Sekanten der Innenkontur des Leitelements 100 darstellen. Die Leitschaufeln 116 erstrecken sich in radialer Einwärtsrichtung bis kurz vor die Vertiefung 112 im Leitelement 100. In radialer Auswärtsrichtung erstrecken sich die Leitschaufeln 116 bis zum äußeren Rand des Leitelements 100. Die Leitschaufeln 116 sind entgegen dem Drall der vom ersten Laufrad 74 abgegebenen Luft angestellt. Sie stellen zugleich Distanzstücke für das Leitelement 100 und den radialen Zwischenboden 108 des Ansaug-Gehäusesegments 106 dar.

Ein Ringraum, der durch die radialen Außenseiten der Umlenkstufenwände 98 und 100, die dortige Umfangswand des Ansaug-Gehäusesegments 106 und die dortigen Bereiche des Zwischenbodens 108 und des Gehäusesegmentes 86 begrenzt ist, ist mit einem Dämmschaum 118 zur Schalldämmung gefüllt.

Oberhalb des Zwischenbodens 108 befindet sich ein Zuströmraum 120, welcher auf der vom Zentrifugalabscheider 14 abgewandten Seite durch einen verrippten Kunststoffdeckel 122 verschlossen ist. Dessen Rippen befinden sich auf der vom Zuströmraum 120 abgewandten Oberseite des Kunststoffdeckels 122.

Der Kunststoffdeckel 122 weist im Zentrum eine kalottenförmige Erhöhung für die Kappe 62 der Rotorglocke 56 des Radialgebläses 12 auf. Auf diese Weise kann die ausströmende Kühlluft der Lager 60 in einem Spalt zwischen dem Kunststoffdeckel 122 und der Kappe 62 vorbei in den Zuströmraum 120 strömen.

Der Kunststoffdeckel 122 verfügt an seiner Umfangsseite über vier kreuzförmig angeordnete Schraublaschen 124, die in Figur 3 gezeigt sind. Die Schraublaschen 124 fluchten in axialer Richtung mit vier entsprechenden Gewindebohrungen 126 in der Trägerplatte 18. Durch jede Schraublasche 124 ist ein in der Figur 3 nicht gezeigter Gewindebolzen durchgesteckt und in der Gewindebohrung 126 der Trägerplatte 18 verschraubt. Auf diese Weise sind das Ansaug-Gehäusesegment 106 und das Gehäusesegment 86 in axialer Richtung zwischen dem Kunststoffdeckel 122 und der Trägerplatte 18 fixiert.

In dem Zuströmraum 120 befindet sich eine etwa kreiszylindrische in Umfangsrichtung unterbrochene Wand 128, welche sich koaxial zur gehäusefesten Achse 16 von der Oberseite des Zwischenbodens 108 nicht ganz bis zur Unterseite des Kunststoffdeckels 122 erstreckt.

In gleichen Abständen weist die Wand 128 etwa 12 in radialer Richtung betrachtet quadratische Durchbrüche 130 auf. Die Durchbrüche 130 erstrecken sich in axialer Richtung nahezu über die gesamte Höhe der Wand 128 und sind auf der dem Zwischenboden 108 zugewandten Seite offen. Die Breite der Durchbrüche 130 in Umfangsrichtung ist etwas kleiner als die Breite der stehengebliebenen Bereiche der Wand 128.

Der Durchmesser der Wand 128 entspricht etwa dem Durchmesser des ersten Laufrads 74. Die Wand 128 hat an einer Umfangsseite eine in radialer Richtung quadratische Einströmöffnung 132, die etwa die Breite der Durchbrüche hat. Die Einströmöffnung 132 ist beidseitig von je einer Platte 134 begrenzt, die jeweils hälftig umfänglich die Wand 128 mit bilden und hälftig senkrecht zur Wand 128 nach außen gebogen sind.

Die Einströmöffnung 132 fluchtet in radialer Richtung mit einem Anschlussstutzen 136, der von der Umfangwand des Ansaug-Gehäusesegmentes 106 getragen ist. Auf den Anschlussstutzen 136 ist von außen ein Verbindungsrohr 138 aufgesteckt. Das Verbindungsrohr 138 kommt von einem Luftauslassstutzen 140 des Zentrifugalabscheiders 14. Ein Ringraum, der durch die Umfangswand des Ansaug-Gehäusesegments 106 im Bereich des Zuströmraums 120 und die Wand 128 begrenzt ist und zwischen der Einströmöffnung 132 und der Öffnung des Anschlussstutzens 136 unterbrochen ist, ist mit Dämmschaum 142 gefüllt. Der Dämmschaum 142 dient der Schalldämmung.

In radialer Richtung zwischen dem Einlasstrichter 112 des ersten Laufrads 74 und der Einströmöffnung 132 ist eine in axialer Sicht V-förmige Strahlteilerwand 144 angeordnet; die Spitze des V zeigt zur Einströmöffnung 132 hin. Die Strahlteilerwand 144 erstreckt sich über die gesamte axiale Höhe des Zuströmraums 120.

Die Strahlteilerwand 144 dient der Aufteilung des aus dem Verbindungsrohr 138 dem Zuströmraum 120 zuströmende Luftstroms in zwei in entgegengesetztem Drall strömende Luftstromteile. Die Zusammenwirkung der Strahlteilerwand 144 mit der die Durchbrüche 130 aufweisenden Wand 128 bewirkt insgesamt eine drallfreie Luftzuströmung in den Einlassstutzen 84 des ersten Laufrads 74.

Ein Abscheider-Antriebsmotor 146 ist analog zum Gebläse-Antriebsmotor 44 aufgebaut. Er weist eine Rotorglokke 148 auf, welche auf dem vom Radialgebläse 12 abgewandten Endabschnitt der gehäusefeste Achse 16 gelagert ist. Die Rotorglocke 148 dreht sich in axialer Richtung zum Zentrifugalabscheider 14 hin gesehen im Uhrzeigersinn.

Der freie Rand der Rotorglocke 148 befindet sich in einem großzügigen Abstand zu einer gedachten Ebene, in der die freien unteren Ränder der Verstärkungsrippen 26 des Trägergehäuses 20 liegen.

Auf der Innenseite der Rotorglocke 148 sind wie bei der Rotorglocke 56 des Gebläse-Antriebsmotors 44 umlaufend Hochleistungsmagnetsegmente aufgeklebt, die in der Figur 1 verdeckt sind.

Entsprechende Statorpakete 150 sind an der radialen Außenseite der Tragbuchse 22 befestigt. Eine Mehrzahl von in Figur 4 schematisch gezeigten Wicklungen 152 der Statorpakete 150 des Abscheider-Antriebsmotors 146 sind über Verbindungsleitungen 154 mit einer Kommutatorplatine 156 und diese über Versorgungsleitungen 158 mit der Steuereinheit 54 verbunden. Die Wicklungen 152 des Abscheider-Antriebsmotors 146 sind mit der Steuereinheit 54 unabhängig von den Wicklungen 46 des Gebläse-Antriebsmotors 44 über getrennte Versorgungskreise unabhängig bestrombar, insbesondere mit unterschiedliche Frequenz aufweisenden Speiseströmen.

Die Kommutatorplatine 156 ist auf der in axialer Richtung der Trägerplatte 18 zugewandten Seite der Statorpakete 148 des Abscheider-Antriebsmotors 146 auf der Tragbuchse 22 befestigt. Sie weist drei Hällgeber 68 für den Abscheider-Antriebsmotor 146 auf und arbeitet entsprechend der Kommutatorplatine 50 des Gebläse-Antriebsmotors 44.

Auf der dem Zentrifugalabscheider 14 zugewandten Stirnseite weist die Rotorglocke 148 des Abscheider-Antriebsmotors 146 ein zur gehäusefesten Achse 16 koaxiales Lagergehäuse 160 auf.

In dem Lagergehäuse 160 sind zwei axial beabstandete Lager 162 angeordnet, welche koaxial zur gehäusefesten Achse 16 von der Tragbuchses 22 gehalten sind.

Die Rotorglocke 148 des Abscheider-Antriebsmotors 146 befindet sich in dem zur gehäusefesten Achse 16 rotationssymmetrischen Elektronikgehäuse 40, welches den gleichen Durchmesser wie die Trägerplatte 18 hat.

Das Elektronikgehäuse 40 ist becherförmig und zum Radialgebläse 12 hin mit der Trägerplatte 18 verschlossen. Der Rand des Elektronikgehäuses 40 sitzt auf dem Rand der Trägerplatte 18 auf und ist mit diesem fest verbunden. Der Boden des Elektronikgehäuses 40 befindet sich zwischen den beiden Lagern 162 des Abscheider-Antriebsmotors 146, nahe dem der Trägerplatte 18 zugewandten Lager 162.

Das Elektronikgehäuse 40 ist aus Metall, so dass es in Verbindung mit dem Trägergehäuse 20 aus Aluminium als Abschirmung gegenüber elektrischen, magnetischen und elektromagnetischen Feldern wirkt.

In radialer Richtung mit dem Rand der Rotorglocke 148 fluchtend ist eine ringförmige Elektronikplatine 164 koaxial zur gehäusefesten Achse 16 angeordnet. Die Elektronikplatine 164 hängt in einem Abstand zu den Verstärkungsrippen 26 des Trägergehäuses 20 an Befestigungszylindern 166 an der Unterseite der Trägerplatte 18. Auf der von der Trägerplatte 18 abgewandten Unterseite trägt die Elektronikplatine 164 eine Vielzahl von gleichermaßen mit dem Bezugszeichen 168 bezeichneten Mikroprozessoren, Leistungshalbleitern und anderen elektronischen Bauelementen, die die Steuereinheit 54 mit bilden. Darüber hinaus weist die Steuereinheit 54 ein in der Figur 4 gezeigtes Netzteil 170 auf, mit dem aus der Netzspannung die erforderlichen Gleichspannungen erzeugt werden können. Zur Steuereinheit 54 führt außerdem ein in der Figur 4 gezeigtes Netzkabel 172.

Im Bereich seines an der Trägerplatte 18 angrenzenden Randes weist das Elektronikgehäuse 40 in seiner Umfangswand eine Vielzahl von Abluftöffnungen 174 auf. Durch die Abluftöffnungen 174 kann erwärmte Luft zur Kühlung der Mikroprozessoren, Leistungshalbleiter und der anderen elektronischen Bauelementen 168 aus dem Elektronikgehäuse 40 abströmen.

Auf der radialen Außenseite des Lagergehäuses 160 des Abscheider-Antriebsmotors 146 ist ein Lüfterrad 176 zur Kühlung des Elektronikgehäuses 40 angebracht. Eine Lüfterradscheibe 178 des Lüfterrads 176 liegt an der dem Zentrifugalabscheider 14 zugewandten Stirnseite der Rotorglocke 148 an. Ihr Durchmesser ist deutlich kleiner als der Durchmesser der Radscheiben 78 der beiden Laufräder 74 und 76.

Die Laufräder 74 und 76, die Rotorglocken 56 und 148 und das Lüfterrad 176 sind jeweils in mindestens zwei Ausgleichsebenen dynamisch ausgewuchtet. Um den Aufwand zu reduzieren, sind die Einzelkomponenten vor der Montage des dentalen Sauggebläses 10 statisch ausgewuchtet.

Auf der von der Rotorglocke 148 abgewandten Seite sind an die Lüfterradscheibe 178 entgegen der Drehrichtung der Rotorglocke 148 konkav gekrümmte Radschaufeln 180 angeformt. Die von der Lüfterradscheibe 178 abgelegenen Ränder der Radschaufeln 180 zeigen nach unten zum Zentrifugalabscheider 14 hin. Die Radschaufeln 180 enden in axialer Richtung knapp über der oberen Seite des Bodens des Elektronikgehäuses 40.

Das Lüfterrad 176 ist von einer umlaufenden, zur gehäusefesten Achse 16 rotationssymmetrischen Leitrippe 182 umgeben, welche etwa die Form der oben erläuterten unteren Umlenkstufenwand 98 aufweist. Die Leitrippe 182 ist einstückig an die obere Seite des Bodens des Elektronikgehäuses 40 angeformt.

Im Zentrum der Leitrippe 182 ist der Boden des Elektronikgehäuses 40 mehrfach treppenartig abgesenkt und bildet dort einen Kühlluftansaugraum 184. Der Kühlluftansaugraum 184 ist auf der Lüfterradseite mit einer Ringplatte 186 verschlossen. Zwischen dem inneren Rand der Ringplatte 186 und der äußeren Umfangsseite des Lagergehäuses 160 der Rotorglocke 148 liegt ein Luftspalt.

Die Umfangswand des Kühlluftansaugraum 184 weist oben nahe der Ringplatte 186 eine Mehrzahl von.Ansaugöffnungen 188 für Kühlluft auf.

Das Lagergehäuse 160 des Abscheider-Antriebsmotors 146 führt unter Beibehaltung eines Laufspiels durch eine Öffnung im Boden des Kühlluftansaugraums 184 in ein Auslaß-Gehäusesegment 190 des Zentrifugalabscheiders 14.

An die Unterseite des Elektronikgehäuses 40 ist außerdem eine Mehrzahl sich in radialer Richtung erstreckender Stabilisierungsrippen 192 angeformt.

Das zur gehäusefesten Achse 16 im Wesentlichen rotationssymmetrische, becherförmige Auslaß-Gehäusesegment 190 ist aus Kunststoff und schließt sich in axialer Richtung an den Kühlluftansaugraum 184 an. Das Auslaß-Gehäusesegment 190 ist oben mit dem Boden des Kühlluftansaugraums 184 dicht verschlossen. Aus dem Auslaß-Gehäusesegment 190 führt seitlich der Luftauslassstutzen 140 heraus, auf dem das Verbindungsrohr 138 steckt.

In die von der Rotorglocke 148 abgewandte offene Stirnseite des Lagergehäuses 160 des Abscheider-Antriebsmotors 146 ist eine zur gehäusefesten Achse 16 koaxiale Antriebsplatte 194 eingesteckt und dort fixiert. An die Antriebsplatte 194 ist eine zur gehäusefesten Achse 16 koaxiale Abscheiderwelle 196 aus rostfreiem Stahl angeformt.

An der Abscheiderwelle 196 sind mehrere Pumpenflügel 198 eines Austragpumpenrades und eine Leitwand 200 einer Zyklonkammer befestigt. Letztere über mehrere radiale Luftrotationsflügel 202, die von der Abscheiderwelle 196 fest getragen sind. Die oben genannten Teile 198, 200 und 202 rotieren somit zusammen mit der Abscheiderwelle 196.

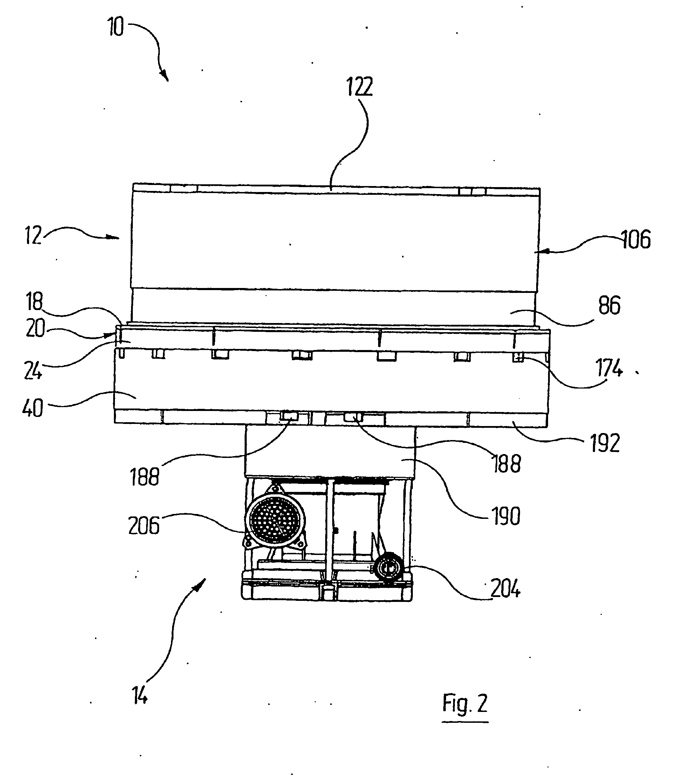

Etwas unterhalb des Auslaß-Gehäusesegments 190 befindet sich gemäß Figuren 2 und 3 in der Umfangswand des Zentrifugalabscheiders 14 ferner ein Eintrittsstutzen 206, an den ein Rohr angeschlossen wird, über das eine Mischung aus Luft und Sekret, welche zu trennen und zu reinigen ist, in den Zentrifugalabscheider 14 geführt wird. Unten weist der Zentrifugalabscheider 14 einen Anschlussstutzen 204 für eine nicht gezeigte Abwasserleitung auf.

Der Zentrifugalabscheider 14 und seine Arbeitsweise ist im Übrigen aus der DE 100 10 077 A1 bekannt.

### Das dentale Sauggebläse 10 arbeitet folgendermaßen:

Mit der Steuereinheit 54 werden die Wicklungen 46 beziehungsweise 152 des Gebläse-Antriebsmotors 44 und des Abscheider-Antriebsmotors 146 separat voneinander bestromt und so die jeweilige Rotorglocke 56 beziehungsweise 148 angetrieben. Der Gebläse-Antriebsmotor 44 wird vorzugsweise auf eine Drehzahl zwischen 10000 bis 14000 Umdrehungen pro Minute geregelt, der Abscheider-Antriebsmotor 146 vorzugsweise auf eine Drehzahl zwischen 3000 bis 4000 Umdrehungen pro Minute.

Der Gebläse-Antriebsmotor 44 treibt dabei die Laufräder 74 und 76 in gleichem Drehsinn und mit gleicher Drehzahl an.

Durch die Drehung des ersten Laufrads 74 wird ein Unterdruck erzeugt, welcher über den Zuströmraum 120 und das Verbindungsrohr 138 in dem Auslaß-Gehäusesegment 190 des Zentrifugalabscheiders 14 wirkt. Die Mischung aus Luft und Sekret wird mit dem über die Steuereinheit 54 vorgegebenen Unterdruck über das Rohr und den Eintrittsstutzen 206 in den Zentrifugalabscheider 14, und von dort in die Zyklonkammer gesaugt.

Durch die Drehung des Abscheider-Antriebsmotors 146 wird über die Abscheiderwelle 196 die Leitwand 200 der Zyklonkammer gedreht, so dass eine Abscheidung des Wassers und der enthaltenen Feststoffe von der Luft in bekannter Weise sowohl nach dem Zyklonprinzip als auch nach dem Zentrifugalprinzip erfolgt.

Das Abwasser mit den enthaltenen Feststoffen wird mit den Pumpenflügeln 198 der Austragpumpe, die über die Abscheiderwelle 196 mit dem Abscheider-Antriebsmotor 146 angetrieben wird, über den Anschlussstutzen 204 in die Abwasserleitung befördert.

Die gereinigte, von festen und flüssigen Bestandteilen freie Luft wird anschließend nach oben in das Auslaß-Gehäusesegment 190 gesaugt. Von dort strömt sie dem Unterdruck folgend über das Verbindungsrohr 138 dem Zuströmraum 120 zu.

Im Zuströmraum 120 wird der Luftstrom mit dem Strahlteilerwand 144 wie bereits beschrieben geteilt, und strömt drallfrei über den Einlassstutzen 84 des ersten Laufrads 74 dem Innenraum dieses Laufrades 74 zu. Die Radschaufeln 80 beschleunigen die Luft nach außen und bewirken so einen Unterdruck an dem Einlassstutzen 84.

Die beschleunigte Luft wird über die Umlenkstufenwände 98 und 100 dem Einlassstutzen 84 des zweiten Laufrads 76 zugeführt, wo sie über die Radschaufeln 80 weiter beschleunigt wird und nach außen in den Auslasskanal 90 geleitet wird. Über den Auslassstutzen 94 wird die Luft unter Druck in die Umgebung geblasen.

Gleichzeitig wird, angetrieben durch den Abscheider-Antriebsmotor 146, mit dem Lüfterrad 176 Kühlluft durch die Ansaugöffnungen 188 im Elektronikgehäuse 40 angesaugt.

Die Kühlluft strömt über die wärmebelasteten Bauteile 168 auf der Elektronikplatine 164 und kühlt diese.

Ein Teil der erwärmten Kühlluft strömt durch die Abluftöffnungen 174 des Elektronikgehäuses 40 ab.

Der Rest der erwärmten Kühlluft strömt durch die Luftschlitze 38 in der Tragbuchse 22 in die Motorkammer 32 des Gebläse-Antriebsmotors 44, die oberhalb der Elektronikplatine 164 liegt.

Die Kühlluft strömt an dem Spalt der Elektronikplatine 164 vorbei zu den Statorpaketen 42 und kühlt diese.

Von da aus strömt die Kühlluft durch die Querbohrung 36 oberhalb der Statorpakete 42 in die Bohrung 34 der gehäusefesten Achse 16.

Die Kühlluft durchströmt den Bohrung 34 in axialer Richtung und kühlt so die beiden Lager 60 des Gebläse-Antriebsmotors 44 von innen her.

Die Kühlluft strömt aus der Stirnseite der gehäusefesten Achse 16 heraus in den Bereich unterhalb der Kappe 62 und von dort durch die Öffnung 64 in den Zuströmraum 120, wo sie sich mit dem oben beschriebenen Hauptluftstrom aus gereinigter Luft vermischt und mit dieser letztendlich zur Umgebung abgeführt wird.

Bei dem oben beschriebenen Ausführungsbeispiel eines dentalen Sauggebläses 10 sind unter anderem folgende Modifikationen möglich:

Die Steuereinheit 54 kann zusätzlich einfach mit optischen und/oder akustischen Ausgabemitteln, beispielsweise mit Warn- und Leuchtanzeigen oder Hupen, verbunden sein. Außerdem kann die Steuereinheit 54 Speichermittel aufweisen, in denen den Betrieb des dentalen Sauggebläses 10 charakterisierende Daten gespeichert werden können, welche beispielsweise Aufschluss über den Betriebsablauf oder die Lebensdauer des dentalen Sauggebläses 10 enthalten können. Diese Daten können beispielsweise zur Reparatur und zu Wartungszwecken abgerufen werden.

Statt die Drehzahlen des Gebläse-Antriebsmotors 44 und/oder des Abscheider-Antriebsmotors 146 jeweils auf eine konstante Drehzahl zu regeln, können mit der Steuereinheit 54 die Drehzahlen auch druckgeführt geregelt werden. Hierzu kann zusätzlich ein Drucksensor vorgesehen sein, mit dem der im Anschlussstutzen 206 herrschende Unterdruck ermittelt und an die Steuereinheit 54 weitergegeben werden kann.

Anstelle einer Motoranordnung, die zwei auf einer gemeinsamen Achse unabhängig laufende Rotoren aufweist, kann man auch zwei unabhängige Motoren verwenden, die axial hintereinanderliegen und über ihre Gehäuse verbunden sind oder deren vorzugsweise becherförmig ausgebildete Gehäuse mit ihren von den Wellen abliegenden Endflächen an den beiden Seiten der Trägerplatte 18 befestigt sind. Solche unabhängige Motoren können dann auch Standard-Innenläufermotoren sein.

## Patentansprüche

1. Saugvorrichtung (10) für dentale, medizinische und industrielle Zwecke mit
a) einem Radialgebläse (12), das eine Saugseite (136) und eine Druckseite (94) aufweist;
b) einem Zentrifugalabscheider (14), dessen Luftauslass (140) mit der Saugseite (136) des Radialgebläses (12) verbunden ist;
c) wenigstens einer Antriebseinheit (44, 146), die zum Antreiben des Radialgebläses (12) und des Zentrifugalabscheiders (14) dient;
d) einer Steuereinheit (54) für die Antriebseinheit (44, 146),
**dadurch gekennzeichnet, dass**
e) die Antriebseinheit einen Gebläse-Antriebsmotor (44) und einen Abscheider-Antriebsmotor (146) umfasst, die als Elektromotoren ausgebildet sind, drehmäßig entkoppelt hintereinander angeordnet sind und das Radialgebläse (12) bzw. den Zentrifugalabscheider (14) antreiben, und
f) der Gebläse-Antriebsmotor (44) und der Abscheider-Antriebsmotor (14) auf einer gemeinsamen gehäusefesten Achse (16) angeordnet sind.

2. Saugvorrichtung nach Anspruch 1, **dadurch gekennzeichnet, dass** die jeweiligen Statoren (42, 150) mit der Achse (16) verbunden sind, die rotierenden Teile (74, 76) des Radialgebläses (12) mit dem Rotor (56) des Gebläse-Antriebsmotors (44) und die rotierenden Teile (198, 200, 202) des Zentrifugalabscheiders (14) mit dem Rotor (148) des Abscheider-Antriebsmotor (146) drehfest verbunden sind und koaxial zur gehäusefesten Achse (16) drehbar gelagert sind.

3. Saugvorrichtung nach Anspruch 2, **dadurch gekennzeichnet, dass** die Rotoren jeweils als Rotorglocke (56, 148) ausgebildet sind, welche jeweils von einem Ende auf die gehäusefeste Achse (16) gesteckt sind und auf ihren Innenflächen die Magentsegmente (66) tragen.

4. Saugvorrichtung nach einem der vorhergehenden Ansprüche, **dadurch gekennzeichnet, dass** der Gebläse-Antriebsmotor (44) und der Abscheider-Antriebsmotor (146) elektronisch kommutiert sind.

5. Saugvorrichtung nach Anspruch 4, **dadurch gekennzeichnet, dass** der Gebläse-Antriebsmotor (44) und der Abscheider-Antriebsmotor (146) je eine Kommutatorplatine (50, 156) mit vorzugsweise je drei Magnetfeldgebern (68) aufweisen und die Kommutatorplatinen (50, 156) auf der gehäusefesten Achse (16) montiert sind.

6. Saugvorrichtung nach einem der vorherigen Ansprüche, **dadurch gekennzeichnet, dass** das Radialgebläse (12) zweistufig ist, wobei vorzugsweise zwei Laufräder (74, 76) hintereinander koaxial mit dem Rotor (56) des Gebläse-Antriebsmotors (44) verbunden sind, die in zu-geordneten Arbeitsräumen (Gehäuse 86, 106) umlaufen, die über wenigstens eine Umlenkstufe (98, 110) miteinander verbunden sind.

7. Saugvorrichtung nach einem der vorherigen Ansprüche, **dadurch gekennzeichnet, dass** ein Lüfterrad (176) zur Kühlung von wärmebelasteten Bauteilen, insbesondere der Steuereinheit (54), der Statoren (42) und/oder von Lagern (60) der Rotoren (56, 148), vorzugsweise zwischen dem Gebläse-Antriebsmotor (44) und dem Abscheider-Antriebsmotor (146) liegend koaxial zur gehäusefesten Achse (16) drehbar angeordnet und mit einem der Antriebsmotoren, insbesondere dem Abscheider-Antriebsmotor (146), antriebsmäßig verbunden ist.

8. Saugvorrichtung nach einem der vorherigen Ansprüche, **dadurch gekennzeichnet, dass** die gehäusefeste Achse (16) wenigstens im Bereich von Lagern (60) einen sich in Längsrichtung erstreckenden Hohlraum (34) aufweist, der über durchgängige Eintrittsverbindungen (36) beziehungsweise Austrittsverbindungen für Kühlluft zur Mantelfläche beziehungsweise zu einer Stirnseite der Achse (16) verfügt.

9. Saugvorrichtung nach einem der vorherigen Ansprüche, **dadurch gekennzeichnet, dass** die Steuereinheit (54) in einem Bereich zwischen dem Gebläse-Antriebsmotor (44) und dem Abscheider-Antriebsmotor (146) angeordnet ist.

10. Saugvorrichtung nach einem der vorherigen Ansprüche, **dadurch gekennzeichnet, dass** die Steuereinheit (54) zwei Versorgungskreise (50, 156) umfaßt, welche den Gebläse-Antriebsmotor (44) und den Abscheider-Antriebsmotor (146) mit unterschiedlicher Drehzahl erregen.

11. Saugvorrichtung nach Anspruch 10, **dadurch gekennzeichnet, dass** der Gebläse-Antriebsmotor (44) und der Abscheider-Antriebsmotor (146) Synchronmotoren sind und ihre Versorgungskreise (50, 156) Speisesignale mit unterschiedlicher Frequenz bereitstellen.

12. Saugvorrichtung nach einem der vorherigen Ansprüche, **dadurch gekennzeichnet, dass** zumindest einer der beiden Antriebsmotoren (46, 146), vorzugsweise beide, eine axiale Abmessung haben, die deutlich kleiner als die radiale Abmessung des Radialgebläses (12) ist, vorzugsweise weniger als 50% nochmals vorzugsweise weniger als 40% derselben beträgt.

13. Saugvorrichtung nach einem der vorherigen Ansprüche, **dadurch gekennzeichnet, dass** die Steuereinheit (54) in einem scheibenförmigen Gehäuseteil (40) untergebracht ist, dessen radiale Abmessung im wesentlichen der des Radialgebläses (12) entspricht.

14. Saugvorrichtung nach einem der vorherigen Ansprüche, **dadurch gekennzeichnet, dass** das Gehäuse des Radialgebläses (12) bei seiner Außenwand zumindest teilweise mit Dämm-Material (96, 118, 142) belegt ist.

## Claims

1. Suction device (10) for dental, medical and industrial purposes with
a) a radial fan (12) which comprises a suction side (136) and a pressure side (94);
b) a centrifugal separator (14), the air outlet (140) of which is connected to the suction side (136) of the radial fan (12);
c) at least one drive unit (44, 146) for driving the radial fan (12) and the centrifugal separator (14);
d) a control unit (54) for the drive unit (44, 146), **characterised in that**
e) the drive unit includes a fan drive motor (44) and a separator drive motor (146), which are realized as electric motors, are arranged in series, rotationally decoupled, and drive the radial fan (12) and the centrifugal separator (14), respectively, and
f) the fan drive motor (44) and the separator drive motor (14) are arranged on a common axle which is fixed with respect to the housing (16).

2. Suction device according to Claim 1, **characterised in that** the respective stators (42, 150) being connected to the axle (16), the rotating parts (74, 76) of the radial fan (12) being connected in non-rotating manner to the rotor (56) of the fan drive motor (44) and the rotating parts (198, 200, 202) of the centrifugal separator (14) being connected in non-rotating manner to the rotor (148) of the separator drive motor (146) and being supported in rotating manner coaxially relative to the axle (16) which is fixed with respect to the housing.

3. Suction device according to Claim 2, **characterised in that** the rotors each take the form of a rotor bell (56, 148) which are each placed from one end onto the axle (16) which is fixed with respect to the housing and carry the magnet segments (66) on their insides.

4. Suction device according to one of the preceding claims, **characterised in that** the fan drive motor (44) and the separator drive motor (146) are electronically commutated.

5. Suction device according to Claim 4, **characterised in that** the fan drive motor (44) and the separator drive motor (146) each exhibit a commutator plate (50, 156) with preferentially three magnetic-field transducers (68) and the commutator plates (50, 156) are mounted on the axle (16) which is fixed with respect to the housing.

6. Suction device according to one of the preceding claims, **characterised in that** the radial fan (12) is in two stages, preferentially two impellers (74, 76) which revolve in assigned working spaces (housings 86, 106) which are connected to one another via at least one deflecting stage (98, 110) being connected in series coaxially to the rotor (56) of the fan drive motor (44).

7. Suction device according to one of the preceding claims, **characterised in that** a ventilator wheel (176) for cooling thermally loaded structural components - in particular the control unit (54), the stators (42) and/or bearings (60) of the rotors (56, 148) - preferentially situated between the fan drive motor (44) and the separator drive motor (146) coaxially relative to the axle (16) which is fixed with respect to the housing is arranged in rotating manner and is connected in driving relationship to one of the drive motors, in particular to the separator drive motor (146).

8. Suction device ordered according to one of the preceding claims, **characterised in that** the axle (16) which is fixed with respect to the housing exhibits, at least in the region of bearings (60), a cavity (34) extending in the longitudinal direction, which is provided with uninterrupted inlet connections (36) and output connections for cooling air to the generated surface and to a front side of the axle (16), respectively.

9. Suction device according to one of the preceding claims, **characterised in that** the control unit (54) is arranged in a region between the fan drive motor (44) and the separator drive motor (146).

10. Suction device according to one of the preceding claims, **characterised in that** the control unit (54) includes two supply circuits (50, 156) which energise the fan drive motor (44) and the separator drive motor (146) at different rotational speeds.

11. Suction device according to Claim 10, **characterised in that** the fan drive motor (44) and the separator drive motor (146) are synchronous motors and their supply circuits (50, 156) provide supply signals with differing frequencies

12. Suction device according to one of the preceding claims, **characterised in that** at least one of the two drive motors (46, 146), preferentially both, have an axial dimension that is distinctly smaller than the radial dimension of the radial fan (12), preferentially amounts to less than 50 %, again preferentially less than 40 % of the same.

13. Suction device according to one of the preceding claims, **characterised in that** the control unit (54) is accommodated in a disc-shaped housing part (40), the radial dimension of which corresponds substantially to t.hat of the radial fan (12).

14. Suction device according to one of the preceding claims, **characterised in that** the housing of the radial fan (12) is covered on its outer wall at least partly with insulating material (96, 118, 142).

## Revendications

1. Dispositif d'aspiration (10) pour usages dentaires, médicaux et industriels, comportant
a) une soufflerie radiale (12) qui présente un côté aspiration (136) et un côté pression (94) ;
b) un séparateur centrifuge (14) dont la sortie d'air (140) est reliée au côté aspiration (136) de la soufflerie radiale (12) ;
c) au moins une unité d'entraînement (44, 146) qui sert à entraîner la soufflerie radiale (12) et le séparateur centrifuge (14) ;
d) une unité de commande (54) de l'unité d'entraînement (44, 146),
**caractérisé en ce que**
e) l'unité d'entraînement comprend un moteur de commande de soufflerie (44) et un moteur de commande de séparateur (146), qui sont réalisés sous forme de moteurs électriques, sont disposés l'un derrière l'autre de façon découplée en rotation et entraînent la soufflerie radiale (12) respectivement le séparateur centrifuge (14), et
f) le moteur de commande de soufflerie (44) et le moteur de commande de séparateur (14) sont disposés sur un axe commun fixe par rapport au carter (16).

2. Dispositif d'aspiration selon la revendication 1, **caractérisé en ce que** les stators respectifs (42, 150) sont reliés à l'axe (16), les pièces en rotation (74, 76) de la soufflerie radiale (12) sont reliées au rotor (56) du moteur de commande de soufflerie (44) et les pièces en rotation (198, 200, 202) du séparateur centrifuge (14) sont reliées au rotor (148) du moteur de commande de séparateur (146) sans pouvoir tourner et sont logées de façon à tourner de façon coaxiale par rapport à l'axe fixe par rapport au carter (16).

3. Dispositif d'aspiration selon la revendication 2, **caractérisé en ce que** les rotors sont respectivement exécutés en tant que cloches de rotor (56, 148) qui sont fixées par une extrémité sur l'axe fixe par rapport au carter (16) et portent sur leurs surfaces internes les segments magnétiques (66).

4. Dispositif d'aspiration selon l'une des revendications précédentes, **caractérisé en ce que** le moteur de commande de soufflerie (44) et le moteur de commande de séparateur (146) sont commutés par voie électronique.

5. Dispositif d'aspiration selon la revendication 4, **caractérisé en ce que** le moteur de commande de soufflerie (44) et le moteur de commande de séparateur (146) présentent respectivement une platine de collecteur, qui comporte de préférence respectivement trois transmetteurs de flux magnétique (68), et **en ce que** les platines de collecteur (50, 156) sont montées sur l'axe fixe par rapport au carter (16).

6. Dispositif d'aspiration selon l'une des revendications précédentes, **caractérisé en ce que** la soufflerie radiale (12) est à deux étages, où de préférence deux roues mobiles(74, 76) sont reliées l'une derrière l'autre, de façon coaxiale, au rotor (56) du moteur de commande de soufflerie (14), lesquelles roues tournent dans des espaces de travail coordonnés (carter 86, 106), qui sont reliés ensemble par au moins un étage de renvoi (98, 110).

7. Dispositif d'aspiration selon l'une des revendications précédentes, **caractérisé en ce qu'**une hélice (176), servant à refroidir les pièces soumises à échauffement, en particulier l'unité de commande (54), les stators (42) et/ou les paliers (60) des rotors (56, 148), est disposée de façon coaxiale par rapport à l'axe fixe par rapport au carter (16) et de façon à pouvoir pivoter, de préférence entre le moteur de commande de soufflerie (44) et le moteur de commande de séparateur (146), et est reliée pour l'entraînement à l'un des moteurs de commande, en particulier au moteur de commande de séparateur (146).

8. Dispositif d'aspiration selon l'une des revendications précédentes, **caractérisé en ce que** l'axe fixe par rapport au carter (16) présente, au moins dans la zone des paliers (60), un espace creux (34) qui s'étend dans la direction longitudinale, et dispose de raccords d'admission traversants (36) ou de raccords de sortie de l'air de refroidissement vers la surface d'enveloppe ou vers une face avant de l'axe (16).

9. Dispositif d'aspiration selon l'une des revendications précédentes, **caractérisé en ce que** l'unité de commande (54) est disposée dans une zone entre le moteur de commande de soufflerie (44) et le moteur de commande de séparateur (146).

10. Dispositif d'aspiration selon l'une des revendications précédentes, **caractérisé en ce que** l'unité de commande (54) comprend deux circuits d'alimentation (50, 156) qui excitent le moteur de commande de soufflerie (44) et le moteur de commande de séparateur (146) avec un régime différent.

11. Dispositif d'aspiration selon la revendication 10, **caractérisé en ce que** le moteur de commande de soufflerie (44) et le moteur de commande de séparateur (146) sont des moteurs synchrones et **en ce que** leurs circuits d'alimentation (50, 156) mettent à disposition des signaux d'alimentation ayant une fréquence différente.

12. Dispositif d'aspiration selon l'une des revendications précédentes, **caractérisé en ce qu'**au moins un des deux moteurs de commande (46, 146), de préférence les deux, a(ont) une dimension axiale qui est considérablement plus petite que la dimension radiale de la soufflerie radiale (12), correspondant de préférence à moins de 50 % et de façon encore plus préférée à moins de 40 % de celle-ci.

13. Dispositif d'aspiration selon l'une des revendications précédentes, **caractérisé en ce que** l'unité de commande (54) est appliquée dans une partie de carter en forme de disque (40) dont la dimension radiale correspond essentiellement à celle de la soufflerie radiale (12).

14. Dispositif d'aspiration selon l'une des revendications précédentes, **caractérisé en ce que** le carter de la soufflerie radiale (12) est recouvert dans sa paroi externe, au moins partiellement, d'un matériau isolant (96, 118, 142).
